# EUROPÄISCHE PATENTSCHRIFT

(11) **EP 0 492 233 B1**
(45) Veröffentlichungstag und Bekanntmachung des Hinweises auf die Patenterteilung: **21.08.1996**
(21) Anmeldenummer: 91121035.9
(22) Anmeldetag: 09.12.1991
(51) Int. Cl.: C07D 263/34

(54) **Verfahren zur Herstellung von 5-Cyano-4-alkyl-oxazol**
Method for the preparation of 5-cyano-4-alkyl-oxazol
Procédé pour la préparation de 5-cyano-4-alkyl-oxazole

(30) Priorität: 21.12.1990 CH 4075/90
(43) Veröffentlichungstag der Anmeldung: 01.07.1992
(73) Patentinhaber: F. HOFFMANN-LA ROCHE AG, 4002 Basel (CH)
(72) Erfinder: Nösberger, Paul, CH-4127 Birsfelden (CH)
(74) Vertreter: Cottong, Norbert A.

(56) Entgegenhaltungen:
- EP-A- 0 010 697
- DE-A- 2 535 310
- DE-A- 2 616 349
- US-A- 3 222 374
- US-A- 4 093 654
- US-A- 4 772 718
- CHEMICAL ABSTRACTS, Band 85, Nr. 19, 8. November 1976, Columbus, Ohio, USA; S.V. STEPANOVA et al.: "Synthesis of 4-methyl-5-cyanooxazole and its reaction with 3-methyl-3-hydroxy-4-penten-1-yne", Seite 518, Spalte 2, Zusammenfassung Nr. 143 020x.

## Beschreibung

Die Erfindung betrifft ein Verfahren zur Herstellung von 5-Cyano-4-C₁₋₆-alkyl-oxazol, insbesondere 5-Cyano-4-ethyl-oxazol und 5-Cyano-4-methyl-oxazol. Diese Oxazole bilden eine wichtige Stoffgruppe. So ist beispielsweise 5-Cyano-4-methyl-oxazol ein wertvolles Zwischenprodukt bei der Synthese von Pyridoxin (Vitamin B₆).

Es wurden bereits einige Verfahren zur Herstellung dieser Oxazole beschrieben. Die Synthese von 5-Cyano-4-methyl-oxazol gelingt beispielsweise durch Dehydratisierung von 5-Carbamoyl-4-methyl-oxazol in Gegenwart von Phosphorpentoxid. Der Nachteil dieses Verfahrens ist jedoch die geringe Produktausbeute, was auf die bei dieser Reaktion sehr leicht auftretende Verkohlung zurückzuführen ist.

Eine Verbesserung dieses Verfahrens gelingt durch die Umsetzung von 5-Carbamoyl-4-methyl-oxazol mit Phosphorpentoxid in Gegenwart von Chinolin als Lösungsmittel (USP 3.222.374). Auch dieses Verfahren hat Nachteile, die sich hauptsächlich aus der Giftigkeit des Chinolins, seinem unangenehmen Geruch sowie der thermischen Instabilität ergeben. Zudem ist Chinolin ein relativ teures Lösungsmittel. Ein weiteres Problem stellt die Regenerierung des Chinolins, die Verwendung von stöchiometrischen Mengen Phosphorpentoxid, die Aufarbeitung der Folgeprodukte des benötigten Phosphorpentoxids, sowie deren umweltgerechte Entsorgung dar.

Ein weiteres bekanntes Verfahren (USP 4.011.234) zur Herstellung des 5-Cyano-4-methyl-oxazols besteht darin, das 5-Carbamoyl-4-methyl-oxazol mit einem Niederalkancarbonsäureanhydrid umzusetzen und das Reaktionsgemisch oder das aus diesem isolierte 4-Methyl-5-(N-niederalkanoyl-carbamoyl)-oxazol einer Pyrolyse zu unterwerfen. Die pyrolytische Endstufe hat jedoch gewisse Nachteile: insbesondere treten Korrosionsprobleme mit den Reaktorwerkstoffen auf, und es bilden sich schwer rezyklisierbare Nebenprodukte.

In der US Patentschrift 4.772.718 wird über die einstufige Ueberführung von 4-Methyl-5-carbonsäureethylester-oxazol in 5-Cyano-4-methyl-oxazol berichtet. Bei diesem Verfahren wird der entsprechende Oxazolester in Gegenwart von Ammoniak und einem Zirkonoxid- oder Hafniumoxidkatalysator in der Gasphase zu dem Cyano-oxazol umgesetzt. Nachteilig ist hier jedoch die Verwendung eines relativ teuren Katalysators sowie - zwecks Erreichung einer optimalen Reaktionsführung - das Einhalten sehr enger Reaktionsbedingungen.

Ein weiteres Verfahren (USP 4.026.901 bzw. DOS 2.616.349) besteht darin, dass 5-Carbamoyl-4-methyl-oxazol katalytisch unter Erhitzen in Anwesenheit von Phosphorpentoxid auf einem festen Träger zu 5-Cyano-4-methyl-oxazol dehydratisiert wird. Nachteilig bei diesem Verfahren sind die Handhabung von 5-Carbamoyl-4-methyl-oxazol, insbesondere die im Vordergrund stehende Sublimation und damit die Feststoffdosierung des schwerflüchtigen Ausgangsmaterials.

Es wurde nun ein Verfahren zur Herstellung von 5-Cyano-4-C₁₋₆-alkyl-oxazol gefunden, das die Nachteile des Standes der Technik nicht aufweist.

Gegenstand der Erfindung ist daher ein Verfahren zur Herstellung von 5-Cyano-4-C₁₋₆-alkyl-oxazol durch Dehydratisierung von 5-Carbamoyl-4-C₁₋₆-alkyl-oxazol in der Gasphase unter Verwendung eines Katalysators auf der Basis von Siliciumdioxid, welches dadurch gekennzeichnet ist, dass 5-Carbamoyl-4-C₁₋₆-alkyl-oxazol in Gegenwart von gegebenenfalls mit Wasser verdünntem N-C₁₋₆-Alkyl-pyrrolidon als inertem Lösungsmittel eingesetzt wird, die resultierende Lösung verdampft und einem Reaktor zugeführt wird, und die Dehydratisierungsreaktion in einem Temperaturbereich von etwa 350°C bis etwa 500°C und bei einem Druck von etwa 50 kPa bis etwa 300 kPa erfolgt.

Als C₁₋₆-Alkylgruppe kommt insbesondere eine Alkylgruppe in Frage, die geradkettig oder verzweigt ist. Die Alkylgruppe ist vorzugsweise geradkettiges C₁₋₃-Alkyl, insbesondere Methyl oder Ethyl.

Als Katalysator kann geformtes Siliciumdioxid (Kugeln, Tabletten, Presslinge usw.) eingesetzt werden. Das Siliciumdioxid kann zur Erhöhung der katalytischen Aktivität und Selektivität mit Oxiden von V, Mo, Fe, Co und/oder Zn belegt werden. Die Belegung kann auf bekannte Weise durchgeführt werden, beispielsweise indem ein in Wasser lösliches Salz des gewünschten Metalls in destilliertem Wasser gelöst wird, das Siliciumdioxid mit dieser Lösung imprägniert wird, und das so imprägnierte Siliciumdioxid getrocknet und bei einer ausreichenden Temperatur (beispielsweise ca. 500°C) calciniert wird.

Die erfindungsgemäss verwendeten Katalysatoren sind im Handel erhältlich oder können auf an sich bekannte Art und Weise selbst hergestellt werden.

Die spezifische Oberfläche des nicht belegten (dotierten) oder mit Oxiden von V, Mo, Fe, Co und/oder Zn dotierten Siliciumdioxid-Katalysators beträgt zweckmässigerweise etwa 20-300 m²/g. Der dotierte Katalysator enthält die Oxide von V, Mo, Fe, Co und/oder Zn zweckmässigerweise in einer Menge von etwa 1-20 Gewichtsprozent, bezogen auf das Gesamtgewicht des dotierten Katalysators.

Eine Verdünnung mit Inertgas, beispielsweise Stickstoff, gegebenenfalls verdünnt mit Wasserstoff zur Reduktion einer eventuell auftretenden Koksablagerung ist zwar möglich, aber für die Durchführung der Reaktion unwesentlich.

Im allgemeinen sollen die Kontaktzeiten im Bereich von etwa 0,1 Sekunde bis etwa 10 Sekunden liegen, wobei der Bereich von etwa 0,3 Sekunde bis etwa 3 Sekunden besonders bevorzugt ist. Die Zufuhr von 5-Carbamoyl-4-niederalkyl-oxazol beträgt pro Liter Katalysator und Stunde zweckmässigerweise ca. 0,1-1,0 kg.

Ferner erfolgt die Umsetzung vorzugsweise bei Temperaturen zwischen etwa 400°C bis etwa 450°C.

Erfindungsgemäss wird das 5-Carbamoyl-4-C₁₋₆-alkyl-oxazol in Form einer Lösung, vorzugsweise einer nahezu gesättigten Lösung, oder einer Suspension in einem N-C₁₋₆-alkyl-pyrrolidon, in den beheizten Reaktor, resp. in das Verdampfungssystem eingeführt, wobei sich eventuell nicht gelöstes 5-Carbamoyl-4-C₁₋₆-alkyl-oxazol spätestens am Siedepunkt im Lösungsmittel löst, und dann zusammen mit dem Lösungsmittel in die beheizte Reaktorzone eingeführt. Das gasförmige Edukt/Lösungsmittelgemisch, eventuell mit einem Inertgas verdünnt, wird durch die beheizte Reaktorzone geführt, die mit dem Katalysator gefüllt ist. Die gasförmigen Produkte werden kondensiert und auf übliche Art und Weise aufgetrennt. Die Ausbeute an 5-Cyano-4-C₁₋₆-alkyl-oxazol beträgt normalerweise etwa 90-95%.

Das verwendete Lösungsmittel sollte vorzugsweise
- so beschaffen sein, dass sich 5-Carbamoyl-4-C₁₋₆-alkyl-oxazol spätestens am Siedepunkt des Lösungsmittels in diesem Lösungsmittel löst
- thermisch stabil und unter den Reaktionsbedingungen inert sein
- einen Siedepunkt besitzen, der höher als der Schmelzpunkt des 5-Carbamoyl-4-C₁₋₆-alkyl-oxazols ist
- leicht von 5-Cyano-4-C₁₋₆-alkyl-oxazol abtrennbar sein
- so beschaffen sein, dass keine Katalysatordesaktivierung auftritt.

Als inerte Lösungsmittel eignen sich insbesondere N-C₁₋₆-Alkyl-pyrrolidone. Ganz bevorzugt wird N-Methylpyrrolidon oder N-Ethylpyrrolidon, gegebenenfalls mit Wasser verdünnt, verwendet.

Das erfindungsgemässe Verfahren wird zweckmässigerweise kontinuierlich durchgeführt, insbesondere in einem Festbettreaktor, der beispielsweise aus einer oder mehreren mit dem Katalysator gepackten Säulen besteht. Der Durchmesser und die Länge der Säulen ist nicht kritisch.

Als Vorteile dieses Verfahrens gegenüber den bisher bekannt gewordenen Verfahren zur Herstellung von 5-Cyano-4-C₁₋₆-alkyl-oxazol sei besonders hervorgehoben, dass
- 5-Carbamoyl-4-C₁₋₆-alkyl-oxazol in Lösung dem beheizten Reaktor zugeführt werden kann
- allenfalls nicht umgesetztes 5-Carbamoyl-4-C₁₋₆-alkyl-oxazol während der Kondensation nicht auskristallisiert, sondern in Lösung bleibt
- die zur Regenerierung des Katalysators notwendige Temperatur ungefähr gleich hoch ist wie die Reaktionstemperatur.

Das erfindungsgemässe Verfahren wird durch folgende Beispiele weiter veranschaulicht:

### Beispiel 1

Der Reaktor besteht aus einem vertikal angeordneten Glasrohr (Länge 45 cm, Durchmesser 2,3 cm), welches von einem elektrisch geheizten Rohrofen umgeben und mit einem Kühler verbunden ist. Der obere Teil des Glasrohrs ist mit 60 ml Keramikkugeln, der mittlere Teil mit 30 ml Katalysator und der untere Teil mit 30 ml Keramikkugeln gefüllt. Als Katalysator werden verschiedene kommerziell erhältliche Siliciumdioxid-Katalysatoren eingesetzt (Tabelle 1).

Die Reaktionszone wird auf 425-450°C aufgeheizt, und es werden dem Reaktor von oben stündlich 30 ml einer 10%igen Lösung von 5-Carbamoyl-4-methyl-oxazol in N-Methyl-pyrrolidon sowie 2 Liter Stickstoffgas zugeführt. Die Reaktionsprodukte werden im Kühler kondensiert und gaschromatographisch analysiert. Die Ergebnisse sind in Tabelle 1 zusammengestellt.

**Tabelle 1**

| **Siliciumdioxid** | **Spezifische Oberfläche (m**^{**2**}**/g)** | **Reaktionstemperatur °C** | **Ausbeute %** |
|---|---|---|---|
| Grace XWP 1000 | 33 | 450 | 94 |
| Grace XWP 500 | 71 | 425 | 93 |
| Shell S980 G | 51 | 450 | 92 |
| BASF D11-11 | 65 | 450 | 83 |
| Akzo Si2-5P | 355 | 425 | 89 |

### Beispiel 2

Als Katalysator wird jeweils ein mit einem Metalloxid belegtes Siliciumdioxid verwendet. Die Belegung des Siliciumdioxids erfolgt auf bekannte Art und Weise: 100 g Siliciumdioxid (BASF D11-11) wird mit der Lösung eines zersetzbaren, in Wasser löslichen Salzes des gewünschten Metalls in 100 ml destilliertem Wasser imprägniert (siehe Tabelle 2). Das belegte Siliciumdioxid wird in einem Rotationsverdampfer bei einem Druck von 2,6 kPa und einer Temperatur von 80°C getrocknet. Anschliessend wird der Katalysator während 3 Stunden bei 500°C (Aufheizrate: 1°C/Min.) in einer Luftatmosphäre calciniert. Die in Tabelle 2 aufgeführten Katalysatoren weisen eine Belegung von 5% Metalloxid auf.

In dem in Beispiel 1 beschriebenen Reaktor wird anstelle des reinen Siliciumdioxids ein mit einem Metalloxid belegter Siliciumdioxid-Katalysator eingefüllt. Die Reaktionszone wird auf etwa 375-425°C aufgeheizt, und es werden dem Reaktor stündlich von oben 30 ml einer 10%igen Lösung von 5-Carbamoyl-4-methyl-oxazol in N-Methyl-pyrrolidon sowie 2 l Stickstoffgas zugeführt.

Die erhaltenen Resultate sind in Tabelle 2 zusammengestellt.

**Tabelle 2**

| **Metalloxid-Salz** | **Menge (g/100 g SiO**_{**2**}**)** | **Metalloxid** | **Reaktionstemperatur °C** | **Ausbeute %** |
|---|---|---|---|---|
| NH₄VO₃ | 6,8 | V₂O₅ | 425 | 88 |
| (NH₄)₆Mo₇O₂₄·4H₂O | 7,3 | MoO₃ | 375 | 89 |
| Fe(NO₃)₃·3H₂O | 26,7 | Fe₂O₃ | 400 | 95 |
| Co(NO₃)₂·6H₂O | 20,5 | CoO | 425 | 91 |
| Zn(NO₃)₂·6H₂O | 19,2 | ZnO | 400 | 94 |

### Beispiel 3

Der Reaktor besteht aus zwei hintereinander geschalteten, von aussen beheizbaren, vertikal angeordneten Stahlrohren (Länge 70 cm, Durchmesser 2,7 cm). Das obere Stahlrohr wird vollständig mit Keramikkugeln gefüllt, das darunter befindliche Rohr wird jeweils am oberen und unteren Ende mit je 150 ml Keramikkugeln gefüllt. Zwischen den Schichten aus Keramikkugeln im unteren Stahlrohr werden 115 ml Siliciumdioxid-Katalysator (Shell S980 G) eingefüllt. Die Temperatur des oberen Stahlrohres wird auf 200°C eingestellt, die Temperatur des unteren Stahlrohres beträgt 460°C. Am Kopf des oberen Rohres werden pro Stunde 330 g einer 10%igen Lösung von 5-Carbamoyl-4-methyl-oxazol in N-Methyl-pyrrolidon zugegeben. Am Fuss des oberen Rohres werden stündlich 100 l Stickstoffgas zugegeben. Insgesamt werden 279g 5-Carbamoyl-4-methyl-oxazol in 2511 g N-Methyl-pyrrolidon innerhalb 8,5 Stunden zudosiert. Am Kopf des Reaktors werden 1828 g reines N-Methyl-pyrrolidon entnommen. Das Reaktionsprodukt wird am unteren Ende des Reaktors als Lösung in N-Methylpyrrolidon entnommen. Der Umsatz beträgt >99%. Bei der destillativen Aufarbeitung des Reaktionsprodukts erhält man 592 g N-Methyl-pyrrolidon, also insgesamt 2420 g (96,4%) des eingesetzten N-Methyl-pyrrolidons. Die Menge des gewonnenen 5-Cyano-4-methyl-oxazols beträgt 222 g (93%).

### Beispiel 4

Anstelle des reinen 5-Carbamoyl-4-methyl-oxazols wird verunreinigtes 5-Carbamoyl-4-methyl-oxazol eingesetzt (Verunreinigung: Wasser, Ammoniak, Ethanol; Gesamtmenge 10%).

Es werden die gleichen Reaktionsbedingungen und die gleichen Mengen an Edukt und Lösungsmittel wie in Beispiel 3 eingesetzt. Die tiefsiedenden Verunreinigungen werden am Kopf des Reaktors entnommen. Die Aufarbeitung des Reaktionsproduktes erfolgt analog Beispiel 3. Die Ausbeute an 5-Cyano-4-methyl-oxazol beträgt 93%.

## Patentansprüche

1. Verfahren zur Herstellung von 5-Cyano-4-C₁₋₆-alkyl-oxazol durch Dehydratisierung von 5-Carbamoyl-4-C₁₋₆-alkyl-oxazol in der Gasphase unter Verwendung eines Katalysators auf der Basis von Siliciumdioxid, dadurch gekennzeichnet, dass das 5-Carbamoyl-4-C₁₋₆-alkyl-oxazol in Gegenwart von gegebenenfalls mit Wasser verdünntem N-C₁₋₆-Alkyl-pyrrolidon als inertem Lösungsmittel eingesetzt wird, die resultierende Lösung verdampft und einem Reaktor zugeführt wird, und die Dehydratisierungsreaktion in einem Temperaturbereich von etwa 350° C bis etwa 500°C und bei einem Druck von etwa 50 kPa bis etwa 300 kPa erfolgt.

2. Verfahren nach Anspruch 1, dadurch gekennzeichnet, dass als 5-Carbamoyl-4-C₁₋₆-alkyl-oxazol 5-Carbamoyl-4-methyl-oxazol oder 5-Carbamoyl-4-ethyl-oxazol verwendet wird.

3. Verfahren nach Anspruch 1 oder 2, dadurch gekennzeichnet, dass als inertes Lösungsmittel gegebenenfalls mit Wasser verdünntes N-Methylpyrrolidon oder N-Ethyl-pyrrolidon verwendet wird.

4. Verfahren nach einem der Ansprüche 1-3, dadurch gekennzeichnet, dass als Katalysator geformtes Siliciumdioxid verwendet wird.

5. Verfahren nach einem der Ansprüche 1-3, dadurch gekennzeichnet, dass mit Oxiden von V, Mo, Fe, Co und/oder Zn dotiertes Siliciumdioxid als Katalysator verwendet wird.

6. Verfahren nach Anspruch 5, dadurch gekennzeichnet, dass die Dotierung von Siliciumdioxid mit Oxiden von V, Mo, Fe, Co und/oder Zn etwa 1-20 Gewichtsprozent beträgt, bezogen auf das Gesamtgewicht des dotierten Katalysators.

7. Verfahren nach einem der Ansprüche 1-6, dadurch gekennzeichnet, dass die Umsetzung in einem Temperaturbereich von ca. 400°C bis ca. 450°C erfolgt.

8. Verfahren nach einem der Ansprüche 1-7, dadurch gekennzeichnet, dass 5-Carbamoyl-4-C₁₋₆-alkyl-oxazol in Form einer Lösung oder einer Suspension in einem N-C₁₋₆-alkyl-pyrrolidon in den beheizten Reaktor resp. in das Verdampfungssystem eingeführt wird, und dann zusammen mit dem Lösungsmittel in die beheizte Reaktorzone, die mit dem Katalysator gefüllt ist, eingeführt wird.

9. Verfahren nach einem der Ansprüche 1-8, dadurch gekennzeichnet, dass pro Liter Katalysator und Stunde ca. 0,1 bis 1,0 kg 5-Carbamoyl-4-C₁₋₆-alkyl-oxazol eingesetzt wird.

10. Verfahren nach einem der Ansprüche 1-9, dadurch gekennzeichnet, dass die Kontaktzeit ca. 0,3 Sekunde bis ca. 3 Sekunden beträgt.

## Claims

1. A process for the manufacture of a 5-cyano-4-C₁₋₆-alkyl-oxazole by dehydration of a 5-carbamoyl-4-C₁₋₆-alkyl-oxazole in the gas phase using a catalyst based on silicon dioxide, characterized in that the 5-carbamoyl-4-C₁₋₆-alkyl-oxazole is used in the presence of a N-C₁₋₆-alkyl-pyrrolidone optionally diluted with water as the inert solvent, the resulting solution is vaporized and fed into a reactor and the dehydration reaction is carried out in a temperature range of about 350°C to about 500°C and at a pressure of about 50 kPa to about 300 kPa.

2. A process according to claim 1, characterized in that 5-carbamoyl-4-methyl-oxazole or 5-carbamoyl-4-ethyl-oxazole is used as the 5-carbamoyl-4-C₁₋₆-alkyl-oxazole.

3. A process according to claim 1 or 2, characterized in that N-methyl-pyrrolidone or N-ethyl-pyrrolidone optionally diluted with water is used as the inert solvent.

4. A process according to any one of claims 1-3, characterized in that formed silicon dioxide is used as the catalyst.

5. A process according to any one of claims 1-3, characterized in that silicon dioxide doped with oxides of V, Mo, Fe, Co and/or Zn is used as the catalyst.

6. A process according to claim 5, characterized in that the doping of silicon dioxide with oxides of V, Mo, Fe, Co and/or Zn amounts to about 1-20 weight percent based on the total weight of the doped catalyst.

7. A process according to any one of claims 1-6, characterized in that the reaction is carried out in a temperature range of about 400°C to about 450°C.

8. A process according to any one of claims 1-7, characterized in that the 5-carbamoyl-4-C₁₋₆-alkyl-oxazole is introduced into the heated reactor or into the evaporation system in the form of a solution or a suspension in a N-C₁₋₆-alkyl-pyrrolidone and is then introduced together with the solvent into the heated reactor zone which is filled with the catalyst.

9. A process according to any one of claims 1-8, characterized in that about 0.1 to 1.0 kg of 5-carbamoyl-4-C₁₋₆-alkyl-oxazole is used per litre of catalyst and hour.

10. A process according to any one of claims 1-9, characterized in that the contact time amounts to about 0.3 second to about 3 seconds.

## Revendications

1. Procédé de préparation de 5-cyano-4-alkyle en C₁ à C₆-oxazole par déshydratation de 5-carbamoyl-4-alkyle en C₁ à C₆-oxazole en phase gazeuse avec utilisation d'un catalyseur à base de dioxyde de silicium, caractérisé en ce qu'on utilise le 5-carbamoyl-4-alkyle en C₁ à C₆-oxazole en présence de N-alkyle en C₁ à C₆-pyrrolidone éventuellement diluée avec de l'eau comme solvant inerte, en ce qu'on vaporise la solution résultante et qu'on l'introduit dans un réacteur, et en ce que la réaction de déshydratation s'effectue dans un intervalle de température allant d'environ 350°C à environ 500°C et à une pression d'environ 50 kPa à environ 300 kPa.

2. Procédé selon la revendication 1, caractérisé en ce qu'on utilise comme 5-carbamoyl-4-alkyle en C₁ à C₆-oxazole le 5-carbamoyl-4-méthyl-oxazole ou le 5-carbamoyl-4-éthyl-oxazole.

3. Procédé selon la revendication 1 ou 2, caractérisé en ce qu'on utilise comme solvant inerte de la N-méthyl-pyrrolidone ou de la N-éthyl-pyrrolidone éventuellement diluée avec de l'eau.

4. Procédé selon l'une des revendications 1-3, caractérisé en ce qu'on utilise comme catalyseur du dioxyde de silicium façonné.

5. Procédé selon l'une des revendications 1-3, caractérisé en ce qu'on utilise comme catalyseur du dioxyde de silicium additionné d'oxydes de V, Mo, Fe, Co et/ou Zn.

6. Procédé selon la revendication 5, caractérisé en ce que l'addition au dioxyde de silicium d'oxydes de V, Mo, Fe, Co et/ou Zn s'élève à environ 1-20% en poids, par rapport au poids total du catalyseur dopé.

7. Procédé selon l'une des revendications 1-6, caractérisé en ce que la réaction est conduite dans un intervalle de température d'environ 400°C à environ 450°C.

8. Procédé selon l'une des revendications 1-7, caractérisé en ce qu'on introduit le 5-carbamoyl-4-alkyle en C₁ à C₆-oxazole sous la forme d'une solution ou d'une suspension dans une N-alkyle en C₁ à C₆-pyrrolidone dans le réacteur chauffé ou dans le système de vaporisation, puis en ce qu'on l'introduit avec le solvant dans la zone chauffée du réacteur remplie de catalyseur.

9. Procédé selon l'une des revendications 1-8, caractérisé en ce qu'on utilise par litre de catalyseur et par heure environ 0,1 à 1,0 kg de 5-carbamoyl-4-alkyle en C₁ à C₆-oxazole.

10. Procédé selon l'une des revendications 1-9, caractérisé en ce que le temps de contact s'élève à environ 0,3 seconde à environ 3 secondes.
